# EUROPEAN PATENT APPLICATION

(11) **EP 4 230 722 A1**
(43) Date of publication of application: **23.08.2023**
(21) Application number: 22157248.0
(22) Date of filing: 17.02.2022
(51) Int. Cl.: C12N 1/20, A61K 35/742

(54) **BACILLOTA STRAINS WITH IMPROVED OUTGROWTH**

(71) Applicant: Evonik Operations GmbH, 45128 Essen (DE)
(72) Inventor: HÜSER, Andrea, 33615 Bielefeld (DE); PELZER, Stefan, 33335 Gütersloh (DE); ERHARDT, Frank, 33604 Bielefeld (DE); GRÜPPEN, Anika, 49835 Wietmarschen (DE); HAKKAART, Xavier, 33615 Bielefeld (DE); DUSCH, Nicole, 33824 Werther (DE); GIATSIS, Christos, 48153 Münster (DE); BORGMEIER, Claudia, 64625 Bensheim (DE); GIRADELLO, Jörg, 69493 Hirschberg (DE); GÜNTHER, Christina, 64342 Seeheim-Jugenheim (DE)
(74) Representative: Evonik Patent Association

(57) **Abstract**

The present invention relates to Bacillus strains with improved outgrowth and/or germination characteristics and their use in feed, food and pharmaceutical compositions.

## Description

The present invention relates to endospore-forming Bacillota strains with improved outgrowth and/or germination characteristics and their use in feed, food and pharmaceutical compositions.

Endospore-forming Bacillota strains are often used as probiotics in the feed and food industry. Their useability as probiotics is due in particular to their ability to inhibit the growth of pathogenic bacteria. But besides that probiotics of the phylum Bacillota often fulfil further functions and are therefore multifunctional feed or food additives with beneficial effects on the health of animals and human beings.

A specific characteristic of the strains which make them useful as probiotics, is their ability to form endospores, which enables them to survive detrimental conditions like, e.g., detrimental conditions occurring during the production of the feed product, in particular the elevated temperatures during the feed pelleting process, or detrimental conditions after take-up of the feed by the animals, in particular the acidic milieu of the stomach. If the milieu becomes more favorable, like the environment of the intestine, the Bacilli are able to transform from endospores into the vegetative state. When they enter the vegetative state, they become metabolically active, produce beneficial substances and are also able to proliferate, which allows to multiply the desired probiotic activity at the target location.

As proliferation of the cells at the target location is one key factor for multiplying the cells' desired activity, like inhibition of pathogenic bacteria, it is of importance to identify the factors and conditions which allow to enhance the cells' ability to proliferate and/or to provide cells which exhibit improved outgrowth and/or germination characteristics at the target location.

According to the invention, it was surprisingly found out that point mutations in the gerKC gene, which encodes a spore germination protein, lead to a significant increase of the cells' ability to outgrow and/or germinate, which is obviously due to an increased activity of the spore germination protein GerKC.

Further, it was in particular also found out that the mutations of the gerKC gene which lead to an increase of the cells' ability to germinate, do not alter the key properties of the parent strain from which the Bacillus cells are derived, above all, no detrimental effects regarding safety and risk aspects were observed and also no negative influence on the probiotic activity, so that the strains with mutations in the gerKC gene are useable as probiotics in the feed and food industry.

The gene gerKC encodes a subunit of the GerK germination receptor (Chen et al. (2014); PLoS One 9(4):e95781). Specific nutrients can bind to germination receptors in the spore's inner membrane and thus trigger the germination of Bacillus spores (Zhang et al. (2013); J Bacteriol. 195(8):1735-40). The germination receptors GerB and GerK are both required together for spore germination, wherein spore germination is triggered by the germinants L-asparagine, D-glucose, D-fructose and potassium ions (AGFK) (Setlow (2003); Curr Opin Microbiol. 6(6):550-6).

The amino acid exchanges in the GerKC protein in the strains according to the invention might lead to a structural change in the protein, enabling a faster reaction upon external germinants and thus leading to a faster germination and outgrowth of the spore. Due to the amino acid exchanges, the protein might be more accessible for the germinant or the activation of the signaling cascade could be triggered more rapidly. The GerKC subunit of the GerK germinant receptor is a lipoprotein, linked to the inner membrane of the spore, whereas the GerKA and GerKB subunits are integral inner membrane proteins. Since the mechanisms of how a germinant is binding to the germination receptor and how the signaling cascade towards germination is started, are still unknown, the exact effect of the amino acid exchanges in the GerKC protein currently cannot be identified with certainty (Christie and Setlow (2020); Cell Signal. 74:109729).

Mutants which show an increased germination due to mutations in the gerKC gene have not been disclosed before.

Thus, a first subject of the present invention, is a endospore-forming Bacillota strain which exhibits improved outgrowth and/or germination characteristics, in particular in comparison to a wild-type and/or parent strain, from which it is derived, wherein the improved outgrowth and/or germination is preferably due to an enhanced activity of the germination receptor GerK, in particular to an enhanced activity of the germination protein KC (GerKC).

Preferably the Bacillota strain has at least one mutation, in particular 1 to 10 mutations, more preferably 1 to 5 mutations, in particular 2 to 4 mutations, above all exactly 2 mutations, in at least one subunit of the germination receptor GerK, in particular in comparison to the receptor subunit sequence of the wild-type and/or parent strain from which it is derived.

More preferably the Bacillota strain has at least one mutation, in particular 1 to 10 mutations, more preferably 1 to 5 mutations, above all 2 to 4 mutations, in particular exactly 2 mutations, in the spore germination protein KC (GerKC), in particular in comparison to the protein sequence of the wild-type and/or parent strain from which it is derived.

According to the invention , the mutated gerKC gene preferably comprises from 1 to 10, more preferably 2 to 5, above all 2 to 4 point mutations, in particular exactly 2 point mutations, in comparison to the gerKC gene of the wild-type strain and/or parent strain, from which it is derived, in particular in comparison to the gerKC sequence of B. velezensis CECT 5940 as disclosed in SEQ ID NO: 1. Accordingly, the mutated GerKC protein preferably comprises from 1 to 10, more preferably form 2 to 5, above all 2 to 4 point mutations, in particular exactly 2 point mutation, in comparison the wild-type and/or parent strain GerKC protein, in particular in comparison to the GerKC protein of *B*. *velezensis* CECT 5940, as disclosed in SEQ ID NO: 2.

In a preferred embodiment of the invention, the protein GerKC contains in the protein sequence glycine at position 11 and/or serine at position 72. In a particularly preferred embodiment of the invention, this is accomplished by the following mutations, in particular starting from a protein which is encoded by the consensus sequence of gerKC as disclosed in SEQ ID NO: 5, above all starting from the GerKC sequence of the strain CECT 5940 as disclosed in SEQ ID NO: 2: substitution of cysteine at position 11 of GerKC by glycine (C11G) and substitution of proline at position 72 of GerKC by serine (P72S), while the coding gene gerKC preferably comprises the following mutations, in particular in comparison to the consensus gerKC sequence as disclosed in SEQ ID NO: 5, above all in comparison to the gerKC sequence of the strain CECT 5940 as disclosed in SEQ ID NO: 1: substitution of thymine at position 31 of gerKC by guanine (T31G) and substitution of cytosine at position 214 by thymine (C214T).

Besides said specific mutation(s) further mutations may be comprised by said sequences, wherein preferably up to 8, more preferably up to 5 or 3, above all 1 or 2 further mutations may be present; but in a very preferred embodiment of the invention, besides that specific one or two mutations as mentioned above, no further mutations are present in the respective sequences, in comparison to the parent and/or wild-type sequences, from which the sequences are derived. In a particularly preferred embodiment, the only difference in the genomic sequence between the parent and/or wild-type strain and the strain according to the invention are said mutations in the gene gerKC.

In a very preferred embodiment of the invention, the gerKC gene has a sequence according to SEQ ID NO: 3 and the GerKC protein has a sequence according to SEQ ID NO: 4.

A further subject of the present invention is accordingly also a protein GerKC which has a sequence identity of at least 95 %, preferably at least 98 or 99 %, to the GerKC sequence of the strain B. velezensis CECT 5940 as disclosed in SEQ ID NO: 2, wherein the protein sequence comprises at least one, preferably both, mutations selected from T31G and C214T. Preferred is a protein GerKC which is encoded by the consensus sequence gerKC according to SEQ ID NO: 5, wherein the protein sequence comprises at least one, preferably both, mutations selected from T31G and C214T. Very preferred is a protein GerKC with a sequence according to SEQ ID NO: 4.

The microorganisms according to the invention can principally be obtained by any kind of method, i.e. by GMO- or non-GMO methods. But preferably the microorganisms are not genetically modified, i.e. non-GMO. This means that the microorganisms are preferably either naturally occurring microorganisms or spontaneous mutants of such naturally occurring microorganisms or microorganisms which are obtained by another method which is classified as non-GMO. The term "spontaneous mutant" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible. Spontaneous mutants might be further, but less preferably, obtained by using mutagens, i.e. chemical substances which induce the formation of mutants. As formation of spontaneous mutants by using mutagens is less preferred, in a preferred embodiment of the invention the spontaneous mutants and/or non-GMO mutants are obtained without the use of such mutagens. If the mutants are obtained by applying gene technological and/or biotechnological methods like site-directed mutagenesis, then preferably methods are applied which are classified as non-GMO. A non-GMO method according to the invention is preferably characterized in that the method does not involve introduction of heterologous genetic information into the microorganism.

In a particularly preferred embodiment of the invention the microorganisms of the invention are naturally non-occurring mutants, in particular non-GMO and/or spontaneous mutants as defined before. The mutants have preferably the same characteristics like the parent strain from which they are derived, with exception of the capacity to outgrow and/or germinate more rapidly.

The microorganisms according to the invention are preferably further non-pathogenic, in particular non-pathogenic to human beings, animals and preferably also non-pathogenic to plants.

The microorganisms according to the invention are preferably selected from a list of microorganisms generally recognized as safe ("GRAS") or listed as direct fed microbials ("DFM") or probiotics as stated in relevant listings, as for example, but not limited to the EFSA QPS list, the AAFCO list or the list by the Chinese Ministery of Agriculture (MOA).

The microorganisms according to the invention have preferably sensitivity for at least five antibiotics, more preferably for at least six, eight or ten antibiotics, above all for at least 11 or 12 antibiotics, wherein the antibiotics are preferably selected from tylosin, nalidixic acid, trimethoprim, apramycin, sulfonamide, ampicillin, vancomycin, gentamicin, kanamycin, streptomycin, erythromycin, clindamycin, tetracycline, chloramphenicol, nourseothricin, daptomycin and virginiamycin. In a very preferred embodiment the microorganisms according to the invention have sensitivity to all antibiotics as mentioned before.

To be "sensitive for antibiotics" means that growth of the microorganism is inhibited by the antibiotic under conditions where the microorganism would otherwise grow. Sensitivity for antibiotics is preferably tested in accordance with the CLSI guidelines (M07-A8 and M45-A2). A Bacillus strain is preferably considered sensitive, if growth is only detected at or below the breakpoint concentration specified in EFSA Journal 2012; 10(6):2740 for vancomycin, gentamicin, kanamycin, streptomycin, erythromycin, clindamycin, tetracycline and chloramphenicol. Concerning tylosin, nalidixic acid, trimethoprim, apramycin, sulfonamide, ampicillin, nourseothricin, daptomycin and virginiamycin, for which no breakpoint is given by EFSA for Bacillus, the breakpoint is preferably 4 mg/L.

The cells of the microorganisms of the invention may be present, in particular in the compositions of the invention, as spores (which are dormant), as vegetative cells (which are growing), as transition state cells (which are transitioning from growth phase to sporulation phase) or as a combination of at least two, in particular all of these types of cells. In a preferred embodiment, the composition of the invention comprises mainly or only spores.

The microorganisms of the invention are of the phylum Bacillota (previously known as Firmicutes), preferably of the genus Bacillus, and are more preferably selected from the species *B*. *subtilis, B. amyloliquefaciens, B. velezensis, B. licheniformis, B. paralicheniformis, B. pumilus, B. megaterium, B. lentus, B. laterosporus, B. alevi, B. cereus, B. badius, B. thurigiensis, B. coagulans, B. siamensis, B. glycinifermentans, B. methylotrophicus, B. thuringensis, B. polyfermenticus, B. vallismortis, B. tequilensis, B. atrophaeus, B. mojavensis, B. sonorensis, B. inaquosus* and *B*. *safensis,* most preferably they are of the species *Bacillus subtilis, Bacillus velezensis, Bacillus amyloliquefaciens, Bacillus pumilus* or *Bacillus megaterium,* above all they are of the species *Bacillus amyloliquefaciens or Bacillus velezensis.*

In a further preferred embodiment of the invention, the microorganisms of the phylum Bacillota are of the genus Clostridium, preferably of the species *C*. *butyricum, C. tyrobutyricum* or *C*. *acetobutylicum.*

In a very preferred embodiment of the invention, the microorganisms according to the invention are of the species *Bacillus velezensis,* more preferably the genomic sequence of the microorganisms is at least 99 %, in particular at least 99.5 % or 99.8 %, above all at least 99.9 % or 99.99 % identical to the genomic sequence of the *B*. *velezensis* strain CECT 5940. In a particularly preferred embodiment, the only difference in the genomic sequence between strain CECT 5940 and the strain according to the invention are said mutations in the gerKC sequence. The strain CECT 5940 is sold under the trademark Ecobiol^{®} by Evonik Operations.

In a very preferred embodiment of the invention, the microorganisms according to the invention additionally possess probiotic activity and/or can be used as direct fed microbials.

The microorganisms according to the invention in particular preferably possess at least one, more preferably at least two, three, four or five, further characteristics selected from:
a) Ability to grow fast in large-scale bioreactors;
b) Ability to grow in the presence of bile, preferably in presence of 2 mM or 4 mM of bile;
c) Ability to grow in the presence of formic acid, acetic acid, propionic acid and/or lactic acid;
d) Ability to produce organic acid(s), in particular lactic acid;
e) Ability to grow under high salt conditions;
f) Ability to form endospores;
g) Ability to grow anaerobically;
h) Ability to inhibit pathogenic bacteria, in particular *C*. *perfringens, S. enteritidis, S. typhimurium, E. coli, C. difficile* and/or *Vibrio parahaemolyticus;*
i) Ability to produce at least one enzyme, preferably selected from amylase, lipase, catalase, cellulase, xylanase and protease;
j) Sensitivity with respect to selected antibiotics;
k) Ability to produce biosurfactants, in particular surfactin;
l) Ability to modulate the microbiome.

The microorganisms of the invention are preferably able to grow fast in large-scale bioreactors. This means in particular heterotrophic growth in the absence of light with doubling rates lower than 60 minutes within stirred tank reactors.

The microorganisms of the invention are preferably further characterized in that they are able to grow in presence of 2 mM bile, preferably in presence of 4 mM bile, in particular characterized by an AUC5 performance value of at least 0.5, preferably at least 0.65, above all at least 0.8, and an AUC10 performance value of at least 1.2, preferably at least 1.4, above all at least 1.6, in presence of 2 mM bile and/or in that they are able to grow in presence of 0.3 wt.-% bile, in particular in presence of 0.3 wt.-% chicken bile and/or in presence of 0.3 wt.-% porcine bile, preferably characterized by being able to survive exposure to 0.3 wt.-% bile, in particular 0.3 wt.-% chicken bile and/or 0.3 wt.-% porcine bile, for at least 3 hours, preferably for at least 5 or 8 hours.

The microorganisms of the invention are preferably further characterized by being able to grow anaerobically, in particular by being able to degrade water-insoluble cellulose and/or protein under anaerobic conditions.

The microorganisms of the invention are preferably further characterized in that at least 50 %, preferably at least 70 or 90 %, of the spores survive exposure to 99°C for 20 minutes.

The microorganisms of the invention are preferably used for preparing feed or food additive compositions by mixing the microorganisms with suitable carriers.

In a particularly preferred embodiment of the invention, the microorganisms of the invention are used in the form of, preferably dried, fermentation broths, wherein "dried" preferably means a residual water content of 0.1 to 10 wt.-%, more preferably 0.2 to 5 wt.-%, above all 0.3 to 4 wt.-%.

A further subject of the present invention is therefore also a feed or food additive containing a microorganism according to the invention, wherein the microorganism may in particular be provided in form of a dried fermentation broth of such a microorganism. The feed or food additive according to the invention preferably comprises the microorganism or a dried fermentation broth thereof in an amount of 0.1 to 10 wt.-%, more preferably in an amount of 0.2 to 5 wt.-%, in particular in an amount of 0.3 to 3 wt.-%. The feed or food additive preferably further comprises a carrier, in particular in an amount of at least 80 wt.-%, preferably in an amount of at least 90 or of at least 95 wt.-%, in particular in an amount of 90 to 99.9 wt.-% or 95 to 99.8 wt.-% or 97 to 99.7 wt.-%. In a preferred embodiment of the invention the feed or food additive consists only of a mixture of the microorganism or a dried fermentation broth of such a microorganism with said carrier.

The feed or food additives preferably contain the microorganism in an amount of 1 × 10⁹ to 1 × 10¹¹ cfu (colony forming units), more preferably in an amount of 5 × 10⁹ to 5 × 10¹⁰ cfu per g feed or food additive.

The carrier as contained in the feed or food additive is preferably selected from inert formulation ingredients added to improve recovery, efficacy, or physical properties and/or to aid in packaging and administration. Such carriers may be added individually or in combination. These carriers may be selected from anti-caking agents, anti-oxidation agents, bulking agents, binders, structurants, coatings and/or protectants. Examples of useful carriers include polysaccharides (in particular starches, maltodextrins, methylcelluloses, gums, chitosan and/or inulins), protein sources (in particular skim-milk powder and/or sweet-whey powder), protein hydrolysates (in particular peptones like soy peptone and/or yeast extract), peptides, sugars (in particular lactose, trehalose, sucrose and/or dextrose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), and silicates (in particular clays, in particular beolite clay, amorphous silica, fumed/precipitated silicas, zeolites, Fuller's earth, Baylith^{®}, clintpolite, montmorillonite, diatomaceous earth, talc, bentonites, and/or silicate salts like aluminium, magnesium and/or calcium silicate). Suitable carriers for animal feed additives are set forth in the American Feed Control Officials, Inc.' s Official Publication, which publishes annually. See, for example Official Publication of American Feed Control Officials, Sharon Krebs, editor, 2006 edition, ISBN 1-878341-18-9. The carriers can be added after concentrating the fermentation broth containing the microorganisms of the invention and/or during and/or after drying of the fermentation broth.

The feed or food additives according to the invention preferably have a residual moisture content of 0.1 to 6 wt.-%, preferably of 0.3 to 5 wt.-%, more preferably of 0.5 to 4 wt.-%. They further preferably exhibit a water activity (a_{w} value) of 0.05 to 0.6, preferably of 0.1 to 0.5.

A further subject of the present invention is also a method of preparing the feed or food additive according to the invention, wherein the microorganisms of the invention or a dried fermentation broth of such microorganisms is mixed with a carrier, in particular as mentioned above, preferably in amount to adjust an amount of 1 × 10⁹ to 1 × 10¹¹ cfu (colony forming units), more preferably to adjust an amount of 5 × 10⁹ to 5 × 10¹⁰ cfu per g feed or food additive.

The feed additives as mentioned before, like the microorganisms itself, can be used for the preparation of feed and pharmaceutical compositions and can be added to drinking and rearing water. In a preferred embodiment 0.1 kg to 100 kg of the feed additive, in particular 1 kg to 10 kg of the feed additive, are used per ton of feed, drinking or rearing water to provide compositions which can be used for feeding animals. The feed and food compositions can be prepared by mixing the feed or food additive with typical feed or food ingredients, respectively.

A further subject of the present invention is therefore also a feed or food composition, containing a microorganism and/or a feed or food additive according to the invention and preferably at least one further feed or food ingredient.

Thus, a further subject of the present invention is also the use of a microorganism of the invention and/or of a feed or food additive of the invention for preparing a feed or food composition.

Thus, a further subject of the present invention is also a method of preparing a feed or food composition according to the invention, wherein a microorganism of the invention and/or a feed or food additive according to the invention are mixed with further feed or food additives.

A further subject of the present invention is also a pharmaceutical composition containing a microorganism according to the invention and at least on pharmaceutically acceptable carrier.

Thus, a further subject of the invention is also the use of a microorganism of the invention and/or of a feed or food additive of the invention for preparing a pharmaceutical composition, in particular a pharmaceutical composition for treating, preventing or mitigating the course of a gut disease.

Thus, a further subject of the present invention is also a method of preparing a pharmaceutical composition according to the invention, wherein a microorganism of the invention and/or a feed or food additive according to the invention are mixed with a pharmaceutically acceptable carrier.

Bacteria of the phylum Bacillota, in particular of the genus Bacillus and of the species *B*. *velezensis,* above all the strain *B*. *velezensis* CECT 5940, are generally known to have a beneficial effect on the gut of animals and human beings.

Thus, the microorganisms of the invention, when administered to animals, preferably enhance the health of such animals and/or improve the general physical condition of such animals and/or improve the feed conversion rate of such animals and/or decrease the mortality rate of such animals and/or increase the survival rate of such animals and/or improve the weight gain of such animals and/or increase the productivity of such animals and/or increase the disease resistance of such animals and/or modulate the immune response of such animals and/or establish or maintain a healthy gut microflora in such animals and/or improve the meat quality of such animals, in particular the meat elasticity and/or the meat hardness, and/or reduce the shedding of bacterial and/or viral pathogens through the feces of such animals. In particular, the microorganisms and compositions of the invention might be used to assist in re-establishing a healthy balance of the gut microflora after administration of antibiotics for therapeutic purposes.

A further subject of the invention is therefore a method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the productivity of animals and/or of increasing the disease resistance of animals and/or of modulating the immune response of animals and/or of establishing or maintaining a healthy gut microflora in animals and/or of improving the meat quality of animals and/or of reducing the shedding of bacterial and/or viral pathogens through the feces of animals, wherein at least one microorganism and/or at least one feed additive and/or at least one composition of the invention is administered to animals.

A further subject of the invention is therefore also a method of enhancing the health of human beings and/or of improving the general physical condition of human beings and/or of increasing the disease resistance of human beings and/or of modulating the immune response of human beings and/or of establishing or maintaining a healthy gut microflora in human beings, wherein at least one microorganism and/or at least one composition according to the invention is administered to human beings.

A further subject of the invention is therefore also the use of at least one microorganism and/or at least one feed additive and/or at least one composition of the invention for (preparing a composition for) enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals, wherein the at least one microorganism and/or at least one feed additive and/or at least one composition of the invention is administered to animals.

A further subject of the invention is therefore also the use of at least one microorganism and/or at least one composition of the invention for (preparing a composition for) enhancing the health of human beings and/or for improving the general physical condition of human beings and/or for increasing the disease resistance of human beings and/or for modulating the immune response of human beings and/or for establishing or maintaining a healthy gut microflora in human beings, wherein the at least one microorganism and/or at least one composition of the invention, is administered to human beings.

A further subject of the invention is therefore also at least one microorganism and/or at least one feed or food additive and/or at least one composition of the invention, for enhancing the health of animals and/or human beings and/or for improving the general physical condition of animals and/or human beings and/or for improving the feed conversion rate of animals and/or for decreasing the mortality rate of animals and/or for increasing the survival rate of animals and/or for improving the weight gain of animals and/or for increasing the productivity of animals and/or for increasing the disease resistance of animals and/or human beings and/or for modulating the immune response of animals and/or human beings and/or for establishing or maintaining a healthy gut microflora in animals and/or human beings and/or for improving the meat quality of animals and/or for reducing the shedding of bacterial and/or viral pathogens through the feces of animals.

"Increasing the productivity of animals" refers in particular to any of the following: production of more or higher quality eggs, milk or meat or increased number of offspring or improved survival of offspring.

By using the microorganisms, feed or food additives and compositions of the invention preferably an improvement of at least one of the features as mentioned before is realized, wherein realization of the feature preferably means an improvement of at least 1 %, more preferably of at least 3 or at least 5 %, in comparison to an adequate negative control. As negative control averages known in the animal husbandry field may be used, but preferably as negative control animals which are subjected to the same treatment like the animals tested are used, but without administration of the microbial preparations and/or compositions of the invention.

In particular, the microorganisms, preparations and compositions of the invention may be administered or fed to an animal in an amount effective to inhibit and/or decrease the growth of pathogenic bacteria in the animal gut. Such pathogenic bacteria include besides *Clostridia, Salmonella, Vibrio* and *Escherichia coli* in particular also *Listeria, Enterococci, Staphylococci, Aeromonas, Streptococci, Campylobacter, Shigella, Haemophilus* and *Brachyspira.* Relatedly, the methods of the present invention may be used to decrease the amount of pathogenic bacteria, viruses and protozoans shed in animal feces. The methods of the present invention may also be used to maintain or increase the growth of beneficial bacteria, such as lactic acid bacteria, in the animal gut. By decreasing pathogenic bacteria and/or increasing or maintaining beneficial bacteria, the compositions of the present invention are able to maintain an overall healthy gut microflora.

Thus, a further subject of the invention is also a method of inhibiting and/or decreasing the growth of pathogenic bacteria and/or for maintaining and/or increasing the growth of beneficial bacteria, in particular in an animal or human gut, wherein the microorganisms, preparations and/or compositions of the invention are administered to animals or humans, and wherein the pathogenic bacteria are preferably selected from *Clostridia,* in particular from *C*. *perfringens, C. difficile, C. novyi, C. septicum* and *C*. *colinum,* from *Listeria,* in particular from *L. monocytogenes, L. seeligeri* and *L. welshimeri,* from *Salmonella,* in particular *S*. *enterica* including the subspecies *enterica, arizonae, bongori* and in particular the serovars, *S*. *gallinarum, S. pullorum, S. typhimurium, S. enteritidis, S. cholerasuis, S. heidelberg, S. dublin, S. hadar, S. typhi, S. paratyphi* and *S*. *infantis,* from *Enterococci,* in particular *E*. *faecalis, E. faecium* and *E*. *cecorum,* from *Staphylococci,* in particular *S*. *aureus,* from *Aeromonas,* from *Streptococci,* in particular *S*. *suis* and *S*. *gallinaceus,* from *Campylobacter,* in particular *C*. *jejuni* and *C*. *coli,* from *Escherichia coli,* from *Haemophilus,* in particular *Haemophilus parasuis,* from *Brachyspira, in particular Brachyspira hyodysenteriae* and from *Vibrio,* in particular *V. parahaemolyticus* and *V. harveyi,* and the beneficial bacteria are preferably selected from lactic acid bacteria, in particular from lactobacilli and bifidobacteria. The pathogenic microorganisms are selected in a preferred embodiment from *Clostridia,* in particular from *C*. *perfringens, Salmonella,* in particular *S*. *enterica,* and *Vibrio,* in particular *V*. *parahaemolyticus.*

In a preferred embodiment of the invention the amount of at least one pathogenic bacterium, in particular the amount of *C. perfringens, S. enterica* and/or *V. parahaemolyticus,* is reduced by at least 0.5 log, more preferably by at least 1 log, 2 log, or 3 log.

Thus, a further subject of the invention are also the microorganisms, preparations and compositions of the invention for inhibiting and/or decreasing the growth of pathogenic bacteria and/or for maintaining and/or increasing the growth of beneficial bacteria, in particular in an animal or human gut, wherein the pathogenic bacteria are preferably selected from *Clostridia,* in particular from *C*. *perfringens, C. difficile, C. novyi, C. septicum* and *C*. *colinum,* from *Listeria,* in particular from *L. monocytogenes, L. seeligeri* and *L. welshimeri,* from *Salmonella,* in particular *S*. *enterica* including the subspecies *enterica, arizonae, bongori* and in particular the serovars, *S*. *gallinarum, S. pullorum, S. typhimurium, S. enteritidis, S. cholerasuis, S. heidelberg, S. dublin, S. hadar, S. typhi, S. paratyphi* and *S*. *infantis,* from *Enterococci,* in particular *E*. *faecalis, E. faecium* and *E*. *cecorum,* from *Staphylococci,* in particular *S*. *aureus,* from *Aeromonas,* from *Streptococci,* in particular *S*. *suis* and *S*. *gallinaceus,* from *Campylobacter,* in particular *C*. *jejuni* and *C*. *coli,* from *Escherichia coli,* from *Haemophilus,* in particular *Haemophilus parasuis,* from *Brachyspira,* in particular *Brachyspira hyodysenteriae* and from *Vibrio,* in particular *V. parahemolyticus* and *V. harveyi,* and the beneficial bacteria are preferably selected from lactic acid bacteria, in particular from lactobacilli and bifidobacteria. The pathogenic microorganisms are selected in a preferred embodiment from *Clostridia,* in particular from *C*. *difficile* and *C*. *perfringens, Salmonella,* in particular *S*. *typhimurium and S. enteritidis, Vibrio,* in particular *V. parahaemolyticus,* and *E. coli.*

The occurrence and/or increased growth of the pathogenic bacteria does or can lead to the outbreak of certain diseases. For example the occurrence and/or increased growth of *Clostridium perfringens* can lead to the outbreak of gut diseases, in particular to the outbreak of necrotic enteritis in swine and poultry. The occurrence and/or increased growth of *C. perfringens* can also lead to the outbreak of further diseases like bacterial enteritis, gangrenous dermatitis and colangiohepatitis. Even the mildest form of infection by *C. perfringens* can already be accompanied by diarrhea, which results in wet litter and by that may lead to secondary diseases like foot pad dermatitis. While *C. perfringens* type C generally is considered to be the primary cause of necrotic enteritis and necrohemorrhagic enteritis in piglets, type A has been linked to enteric disease in suckling and feeding pigs with mild necrotic enterocolitis and villous atrophy.

*Clostridium difficile* is an important emerging pathogen that causes diarrhea primarily in neonatal swine. Affected piglets may have dyspnea, abdominal distention, and scrotal edema.

*Staphylococcus aureus subsp. aureus* can cause bumblefoot in chickens, streptococcal mastitis in sows and it is capable of generating toxins that produce food poisoning in the human body.

*Salmonella* bacteria are an important cause of food poisoning of humans, which often is linked to the consumption of meat, such as poultry meat, pork or products derived therefrom. Accordingly, controlling *Salmonella* is a significant challenge for the meat-producing industry. In Europe, a baseline study conducted in 2005 on the prevalence of *Salmonella* in egg-laying flocks has shown that at the global EU-level, 20.3% of the large-scale laying hen holdings are bacteriologically positive for S. *enteritidis.* In some countries, the prevalence was even higher than 80% [European Food Safety Authority (2006), "Preliminary report: analysis of the baseline study on the prevalence of salmonella in laying hen flocks of Gallus gallus*"*]*.*

*Vibrio parahaemolyticus* is in particular responsible for diseases of crustaceans like the Early Mortality Syndrome (EMS), also known as Acute Hepatopancreatic Necrosis Disease (AHPND), which affects both Giant Tiger Prawn (*Penaeus monodon*) and Whiteleg Shrimp (*Penaeus vannamei*)*.*

Pathogens can cause further diseases like polyarthritis, fibrinous polyserositis, post-weaning enteric disorders like post-weaning diarrhea and edema disease and swine dysentery.

A preferred subject of the invention is therefore a therapeutic composition for treating a disease, in particular for treating a gut disease, in particular related to bacterial infection by Clostridia, in particular by *C*. *difficile* or *C*. *perfringens,* and/or by Salmonella, in particular *S*. *enterica,* preferably *S*. *enterica subsp. enterica enteritidis,* or *S*. *typhimurium,* and/or by Vibrio, in particular *Vibrio parahaemolyticus* and/or by *E*. *coli.*

A further preferred subject in this context is therefore a therapeutic composition for treating and/or preventiing necrotic enteritis and/or necrohemorrhagic enteritis, in particular sub-clinical necrotic enteritis and/or necrohemorrhagic enteritis, in animals, preferably swine or poultry, comprising the strains and/or preparations and/or compositions of the invention.

A further preferred subject in this context is therefore a therapeutic composition for treating and/or preventing the Early Mortality Syndrome in animals, preferably animals kept in aquaculture, more preferably crustaceans, in particular shrimps and prawns, comprising the microorganisms and/or preparations and/or compositions of the invention.

A further preferred subject in this context is therefore a therapeutic composition for treating and/or preventing diseases caused by food poisoning, comprising the microorganisms and/or preparations and/or compositions of the invention.

Another preferred subject in this context is therefore a therapeutic composition for treating and/or preventing of bacterial enteritis, gangrenous dermatitis, colangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, in animals, preferably in swine or poultry, comprising the microorganisms and/or preparations and/or compositions of the invention.

A further subject of the invention is therefore also the treatment and/or prevention of a disease, in particular of a gut disease, related to bacterial infection by Clostridia, in particular *C. perfringens,* and/or by Salmonella, in particular *S. enterica,* preferably *S. enterica subsp. enterica enteritidis,* and/or by Vibrio, in particular *Vibrio parahaemolyticus,* wherein a microorganism and/or preparation and/or composition of the invention is administered to an animal in need thereof, wherein the animal is preferably swine or poultry.

A further subject of the invention is therefore also the treatment and/or prevention of a disease, in particular of a gut disease, preferably of necrotic enteritis or necrohemorrhagic enteritis, in particular of sub-clinical necrotic enteritis or sub-clinical necrohemorrhagic enteritis, wherein a microorganism and/or preparation and/or composition of the invention is administered to an animal in need thereof, wherein the animal is preferably swine or poultry.

A further subject of the invention is therefore also the treatment and/or prevention of a disease of aquatic animals, in particular of the Early Mortality Syndrome, wherein a microorganism and/or preparation and/or composition of the invention is administered to an animal in need thereof, wherein the animal is preferably an animal kept in aquaculture, more preferably crustaceans, in particular shrimps and prawns.

A further subject of the invention is therefore also the treatment and/or prevention of diseases caused by food poisoning, wherein a microorganism and/or preparation and/or composition of the invention is administered to an animal or human being in need thereof.

A further subject of the invention is therefore also the treatment and/or prevention of diseases caused by food poisoning, wherein a microorganism and/or preparation and/or composition of the invention is applied to an animal product, in particular to meat or eggs, to avoid the poisoning of human beings.

A further subject of the invention is therefore also the treatment and/or prevention of a disease, preferably a disease of swine or poultry, selected from bacterial enteritis, gangrenous dermatitis, colangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, wherein a microorganism and/or preparation and/or composition of the invention is administered to an animal in need thereof.

The methods and uses of the microorganisms, feed or food additives and compositions of the invention can be pharmaceutical or non-pharmaceutical, in particular therapeutic or non-therapeutic. In a particularly preferred embodiment of the invention, the methods and uses are non-pharmaceutical, in particular non-therapeutic, preferably feed and food applications.

Preferred food compositions according to the invention are fermented foods and/or dairy products, in particular yoghurt, cheese, milk, butter, curd and natto. The microorganisms according to the invention may in particular be used in beverages and in milk replacers, for examples in a baby formula.

The pharmaceutical compositions according to the invention preferably contain at least one pharmaceutically acceptable carrier. They may further contain other ingredients like typical food ingredients as listed further below.

The pharmaceutical composition according to the invention is preferably a liquid, a paste, an aerosol, a suppository or a dried composition, in particular to be administered to the pharyngeal/respiratory tract, to the gastro-intestinal tract, to the urogenital tract or to the skin. Administration might be carried out by using a medical device, for example a spray, a pump or an inhalator.

Thus, a further subject of the present invention is also a method of preparing a pharmaceutical composition, wherein microorganisms according to the invention are mixed with a pharmaceutically acceptable carrier and optionally further compounds and wherein the pharmaceutical composition may be provided in the form of a medical device.

A further subject of the present invention is in particular also a nutritional supplement containing microorganisms according to the invention and at least one pharmaceutically acceptable carrier.

In a preferred embodiment of the invention, the microorganisms and compositions of the invention are administered orally to animals or human beings. The administration is preferably carried out in form of feed and food compositions, but may also be carried out by applying the microbial preparations or feed or food additives of the invention to drinking or rearing water. Thus, a further subject of the present invention is water, in particular an aqueous solution or an aqueous suspension, containing at least one microorganism or feed or food additive according to the invention.

In particular, when they are administered to drinking or rearing water, the microorganisms and compositions of the invention may be applied in the form of compositions, in particular tablets, which after exposure to the drinking or rearing water dissolve easily and set free the microorganisms into the aqueous environment. Such compositions, in particular tablets, are a further preferred embodiment of the invention. Those compositions preferably comprise carriers like betaine salts, citrates, carbonates and/or bicarbonates like for example disclosed in WO 2020/225020.

The feed, food and pharmaceutical compositions of the invention as well as the drinking water, rearing water, effluent water or wastewater, preferably comprise microorganisms at a rate of about 1×10² to about 1×10¹⁰ CFU/g feed or ml water, preferably in an amount of about 1×10⁴ to about 1×10⁹ CFU/g feed or ml water, more preferably in an amount of 1×10⁶ to 1×10⁸ CFU/g feed or ml water.

Correspondingly, preferred amounts of feed or food additive of the invention in the feed, food and water compositions of the invention range from 0.01 wt.-% to 10 wt.-%, more preferably from 0.05 wt.-% to 5 wt.-%, in particular from 0.1 wt.-% to 2 wt.-%.

The feed, food and pharmaceutical compositions of the invention may comprise additional carriers or further typical feed ingredients or combinations thereof.

Suitable carriers for the preparation of feed, food and pharmaceutical compositions are the same as mentioned before for the preparation of the feed or food additive starting from the microorganism of the invention or a fermentation broth of such microorganisms.

Suitable typical animal feed ingredients which may be contained in the compositions according to the invention and/or used in the preparation of feed or food compositions starting from the microorganisms and/or microbial fermentation broths and/or feed or food additives according to the invention include one or more of the following: proteins, carbohydrates, fats, further probiotics, prebiotics, enzymes, vitamins, immune modulators, milk replacers, minerals, amino acids, carriers, coccidiostats, acid-based products and/or medicines, such as antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

The feedstuff according to the invention has preferably a total protein content of 20 to 50 wt.-% and/or a total fat content of 1 to 15 wt.-% and/or a total carbohydrate content of 20 to 60 wt.-%.

Further probiotics (DFM) and/or microorganisms which may be used according to the invention in combination with the microorganisms and feed additives of the invention are preferably bacteria selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Enterococcus faecium,* and *Pediococcus acidilactici.* Preferred examples are *Bacillus pumilus* DSM 32539 and *Bacillus subtilis* DSM 32540 (both deposited with the DSMZ on June 14, 2017 under the provisions of the Budapest Treaty on the International Recognition of the Deposit of Microorganisms for the Purpose of Patent Procedure) and derivatives thereof, *Bacillus licheniformis* DSM 32314 and *Bacillus subtilis* DSM 32315 (both deposited with the DSMZ on May 12, 2016 under the provisions of the Budapest Treaty on the International Recognition of the Deposit of Microorganisms for the Purpose of Patent Procedure) and derivatives thereof, *Bacillus subtilis* PB6 (as described in US Patent No. 7,247,299 and deposited as ATCC Accession No. PTA-6737), which is sold by Kemin under the trademark CLOSTAT^{®}, *Bacillus subtilis* C-3102 (as described in US Patent No. 4,919,936 and deposited as FERM BP- 1096 with the Fermentation Research Institute, Agency of Industrial Science and Technology, in Japan), sold by Calpis as CALSPORIN^{®}, *Bacillus subtilis* DSM 17299, as sold by Chr. Hansen under the trademark GalliPro^{®}, *Bacillus licheniformis* DSM 17236, as sold by Chr. Hansen under the trademark GalliProTect^{®}, a mixture of *Bacillus licheniformis* DSMZ 5749 and *Bacillus subtilis* DSMZ 5750 spores, as sold by Chr. Hansen under the trademark BioPlus^{®}YC, *B*. *subtilis* DSM 29784, as sold by Adisseo/Novozymes under the trademark Alterion^{®}, *Bacillus subtilis,* as sold by Chr. Hansen under the trademark PORCBOOST^{®}, *B. licheniformis* DSM 28710, as sold by Hevepharma under the trademark B-Act^{®}, a three-strain *Bacillus* probiotic, as sold by DuPont under the trademark Enviva^{®} PRO, the probiotic PureGro^{™}, as sold by DSM, or *Bacillus coagulans* microorganisms as described in US Patent No. 6,849,256. Other non-Bacillus probiotics, such as *Saccharomyces cerevisiae, Pichia pastoris, Aspergillus niger, Aspergillus oryzae,* or *Hansenula,* may also be used in compositions of the present invention. In particular in food compositions further probiotics which are known to be useful to the human health may be used such as lactic acid producing bacteria of the order Lactobacillales, more preferably of the families Aerococcaceae, Carnobacteriaceae, Enterococcaceae, Lactobacillaceae, Leuconotocaceae or Streptococcaceae, or of the order Bifidobacteriales, preferably of the family Bifidobacteriaceae, in particular of the genus Bifidobacterium. If said further probiotics are not formulated as part of the compositions of the present invention, they may be administered together (either at the same time or at different times) with the compositions of the present invention.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used in feed compositions according to the invention and which may aid in the digestion of feed, are preferably selected from phytases (EC 3.1 .3.8 or 3.1.3.26), xylanases (EC 3.2.1.8), galactanases (EC 3.2.1 .89), galactosidases, in particular alpha-galactosidases (EC 3.2.1.22), proteases (EC 3.4), phospholipases, in particular phospholipases A1 (EC 3.1 .1.32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ); lysozymes (EC 3.2.1 .17), glucanases, in particular beta-glucanases (EC 3.2.1.4 or EC 3.2.1.6), glucoamylases, cellulases, pectinases, or any mixture thereof.

Examples of commercially available phytases include Bio-Feed^{™} Phytase (Novozymes), Ronozyme^{®} P and HiPhos^{™} (DSM Nutritional Products), Natuphos^{™} (BASF), Finase^{®} and Quantum^{®} Blue (AB Enzymes), the Phyzyme^{®} XP (Verenium/DuPont) and Axtra^{®} PHY (DuPont). Other preferred phytases include those described in e.g. WO 98/28408, WO 00/43503, and WO 03/066847.

Examples of commercially available xylanases include Ronozyme^{®} WX and G2 (DSM Nutritional Products), Econase^{®} XT and Barley (AB Vista), Xylathin^{®} (Verenium) and Axtra^{®} XB (Xylanase/beta-glucanase, DuPont). Examples of commercially available proteases include Ronozyme^{®} ProAct (DSM Nutritional Products).

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Immune modulators which may be used are for example antibodies, cytokines, spray-dried plasma, interleukins, or interferons, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof.

Amino acids which may be used according to the invention are for example lysine, alanine, threonine, methionine or tryptophan, or combinations thereof.

The compositions of the invention may further comprise betaine and/or choline and/or other physiologically effective methyl group donors. The feedstuffs may further comprise polyunsaturated fatty acids, in particular DHA and/or EPA.

Thus, a further embodiment of the invention is also a method of preparing an animal feed composition comprising mixing at least one microorganism and/or microbial preparation and/or feed additive of the invention, in particular in an amount effective to enhance animal health, in particular gut health, with feed ingredients, such as proteins, lipids and/or carbohydrates, and optionally further beneficial substances, preferably as mentioned before, to provide a feeding product. This method may comprise for example also a pelleting step.

Standard pelleting processes known to those of skill in the art may be used, including extrusion processing of dry or semi-moist feeds. Preferred pelleting temperatures are between about 65° C and about 120° C.

The microorganisms, microbial preparations, feed additives and compositions of the invention can be administered to animals in feed and/or drinking water and/or rearing water over multiple days throughout the animal's life or during particular stages or portions of the animal's life. For example, the microorganisms and/or microbial preparations and/or compositions can be administered only in a starter diet or only in a finisher diet of farm animals.

The microorganisms, microbial preparations and compositions of the invention can further be employed in a wide dosage range. Daily doses are, for example, in the range of between approximately 1 mg and approximately 500 mg, in particular in the range of approximately 5 mg and approximately 200 mg, in the range of from approximately 10 mg to approximately 100 mg or in the range of from approximately 20 to approximately 60 mg per kilogram live weight.

Preferably according to the invention always an effective amount of the microorganisms and/or microbial preparations and/or feed or food additives and/or compositions of the invention is used in the embodiments of the invention. The term "effective amount" refers to an amount which effects at least one beneficial effect to an animal and/or to the environment, in particular with respect to the features as already mentioned before, in comparison to an animal or environment that has not been administered the microorganisms and/or microbial preparations and/or compositions of the invention, but besides that has been administered the same diet (including feed and other compounds) or the same composition, respectively.

In case of therapeutic applications preferably a therapeutic amount of the microorganisms and/or microbial preparations and/or feed or food additives and/or compositions of the invention is used. The term "therapeutic amount" refers to an amount sufficient to ameliorate, reverse or prevent a disease state in an animal. Optimal dosage levels for various animals can easily be determined by those skilled in the art, by evaluating, among other things, the composition's ability to (i) inhibit or reduce pathogenic viruses and/or bacteria in the gastrointestinal tract, in particular in the gut, or in the oral or nasopharyngeal cavity or on the skin or in the gills at various doses, (ii) increase or maintain levels of beneficial bacteria and /or (iii) enhance animal health, in particular gut health, at various doses.

The methods of the present invention may be used for all kinds of animals, in particular all kinds of vertebrates such as mammals, aquatic animals and birds.

Animals that may benefit from the invention include but are not limited to farm animals, pets, exotic animals, zoo animals, animals used for sports, recreation or work.

In a very preferred embodiment of the invention, the animals are farm animals, which are raised for consumption or as food-producers, such as poultry, swine and ruminants.

The poultry may be selected from productive or domestic poultry, but also from fancy poultry or wild fowl. Preferred productive poultry in this context are chickens, turkeys, ducks and geese. The productive livestock in this context is preferably poultry optimized for producing young stock or poultry optimized for bearing meat. Preferred fancy poultry or wild fowl are peacocks, pheasants, partridges, chukkars, guinea fowl, quails, capercaillies, grouse, pigeons and swans, with quails being especially preferred. Further preferred poultry are ratites, in particular ostriches and emus, as well as parrots.

Ruminants according to the invention are preferably selected from cattle, goat and sheep. In one embodiment, the compositions of this invention may be fed to preruminants to enhance their health and, in particular, to decrease the incidence of diarrhea in these animals. Preruminants are ruminants, including calves, ranging in age from birth to about twelve weeks.

Pets are preferably selected from dogs, cats, domestic birds and domestic exotic animals. Animals used for sports may in particular be horses.

The aquatic animals according to the invention are preferably finfish, in particular of the class Actinopterygii, or crustaceans. Actinopterygii include, in particular, tilapia and other cichlids, carps and other cyprinids, salmons and other salmonids, catfish, in particular African catfish and pangasius, tuna, perch, cod, smelt, milkfish, gourami, seabass, in particular barramundi, seabream, grouper and snakehead fish.

Preferred types of salmon and salmonids in this context are the Atlantic salmon, red salmon (sockeye salmon), masu salmon (cherry salmon), king salmon (Chinook salmon), keta salmon (chum salmon), coho salmon, Danube salmon, Pacific salmon, pink salmon and trout. The aquatic animals according to the invention are very preferred tilapia.

Crustaceans include in particular shrimps, lobster, crabs, prawns and crayfish.

Preferred types of shrimps in this context are *Litopenaeus, Farfantepenaeus* and *Penaeus,* in particular *Penaeus stylirostris, Penaeus vannamei, Penaeus monodon, Penaeus chinensis, Penaeus occidentalis, Penaeus calif omiensis, Penaeus semisulcatus, Penaeus esculentu, Penaeus setiferus, Penaeus japonicus, Penaeus aztecus, Penaeus duorarum, Penaeus indicus,* and *Penaeus merguiensis.* Very preferred according to the invention is the shrimp *Penaeus vannamei,* also called whiteleg shrimp.

The aquatic animals, and in particular the shrimp, which are treated or fed with the microorganisms and/or preparations and/or compositions according to the invention can in particular be larvae, post-larvae or juvenile shrimp.

The aquatic animals may in particular also be fish which is subsequently processed into fish meal or fish oil. In this connection, the fish are preferably herring, pollack, cod or small pelagic fish like anchovy, blue whiting, capelin, driftfish, jack, mackerel, menhaden, sardine or scad fish. The fish meal or fish oil thus obtained, in turn, can be used in aquaculture for farming edible fish or crustaceans.

The aquatic animals may further be oysters, clams, cockles, arkshells, bivalves, mussels or scallops.

However, the aquatic animals may also be small organisms which are used as feedstuff in aquaculture. These small organisms may take the form of, for example, nematodes, small crustaceans or rotifers.

The farming of aquatic animals may take place in ponds, tanks, basins or else in segregated areas in the sea or in lakes or in rivers, in particular in this case in cages or net pens. Farming may be used for farming the finished edible fish, but also may be used for farming fry which are subsequently released so as to restock the wild fish stocks.

In salmon farming, the fish are preferably first grown into smolts in freshwater tanks or artificial watercourses and then grown on in cages or net pens which float in the sea, ponds or rivers and which are preferably anchored in bays or fjords.

Accordingly, the feed composition according to the invention is preferably a feedstuff for use in the farming of the above-mentioned animals. Preferred applications for animal feed according to the invention is in the feed of cattle, swine, poultry, fish, crustacean or companion animals, with swine, in particular weaned piglets, and poultry being particularly preferred. Spore forming microorganisms, in particular of the genus *Bacillus,* are particularly suited for this application as they produce heat resistant spores which can be mixed into feed prior to the pelleting process of animal feed. Microorganisms according to the invention are very preferably used in milk replacers for cattle, goats, sheep and swine or as post-pelleting spray-on coating for pelleted feed material.

The microorganisms and fermentation broths to be used according to the present invention can be obtained by culturing the microorganisms of the invention according to methods well known in the art, including by using the media and other methods as described for example by Elisashvili et al. (Probiotics and Antimicrobial Proteins 11, 731-747 (2019)), Ren et al. (AMB Express 8:21 (2018)) or as described in the Report 179 of the Food and Agriculture Organization of the United Nations (FAO) with the title "Probiotics in Animal Nutrition" (Bajagai et al., FAO (2016)). Conventional large-scale microbial culture processes include submerged fermentation, solid state fermentation, or liquid surface culture. Fermentation is configured to obtain high levels of colony forming units of the microbial cells. Optionally, the fermentation broth thus obtained may be washed, for example via a diafiltration process, to remove residual fermentation broth and metabolites. Fermentation broths as used for the preparation of feed or food additives according to the invention preferably contain, after the fermentation is finished, cells in an amount of 5 × 10¹⁰ to 5 × 10¹², more preferably in an amount of 1 × 10¹¹ to 4 × 10¹² cfu/g.

### Working examples

### Example 1: Comparison of genomic DNA of the mutated strain with the parent strain CECT 5940

The parent strain CECT 5940 was deposited in April 2001 in the Spanish Culture Collection CECT and is sold as probiotic product for many years. Originally it was classified as *B*. *amyloliquefaciens* strain, but it was reclassified recently as *B. velezensis* strain. A sequence comparison of the mutated strain by mapping showed that the genomic DNA of the mutated strain contained only two point mutations in the gene sequence which encodes the germination protein KC (GerKC) and was otherwise identical. The two point mutations which are contained in the mutated gene lead to an exchange of cysteine at position 11 of GerKC to glycine (C11G) and to an exchange of proline at position 72 of GerKC to serine (P72S).

### Example 2: Comparison of morphological features of the mutated strain and the parent strain

Bacillus CECT 5940 is described as an aerobic spore-forming, Gram-positive Bacillus. Their vegetative cells are rod-shaped (0.7 - 0.9 µm length × 1.8 - 3.0 µm width), very often grouped in chains. They are slightly mobile, with peripheral flagella. The spores are of ellipsoidal shape, with no sporangic deformation. The two point mutations in the germination receptor subunit gerKC do not have any influence on the phenotypic appearance of the strain and on its cultivation conditions.

### Example 3: Comparison of antimicrobial susceptibility of the mutated strain and the parent strain

Bacillus CECT 5940 strain has been found to be susceptible to chloramphenicol, clindamycin, daptomycin, erythromycin, gentamicin, kanamycin, streptomycin, nourseothricin, tetracycline, vancomycin and virginiamycin M1. The phenotypic antimicrobial resistance is not changed by the genetic modifications in the gerKC gene. Both strains are susceptible to the tested antibiotics.

### Example 4: Comparison of production of undesired antimicrobials

To assess the production of antimicrobials relevant for the therapeutic use in humans and animals in accordance with the EFSA guidance (EFSA, 2018) the inhibitory activity of culture supernatants of Bacillus CECT 5940 against the reference strains Bacillus subtilis ATCC 6633, Enterococcus faecalis ATCC 29212, Escherichia coli ATCC 25922, Pseudomonas aeruginosa ATCC 27853 and Staphylococcus aureus ATCC 25923 was determined. No inhibition was observed for both the Bacillus CECT 5940 wildtype denoting the lack of antimicrobial production, including aminoglycosides. The lack of the production of antimicrobials relevant for the therapeutic use in humans and animals is not changed by the genetic modifications in the gerKC gene of Bacillus CECT 5940.

### Example 5: Comparison of toxigenicity of the mutated strain and the parent strain

A cytotoxicity study on Vero C1008 epithelial cells has confirmed that Bacillus CECT 5940 wildtype lacks toxigenic potential and this is also true for the mutated strain. No cytotoxicity was observed for the negative control, for Bacillus CECT 5940 and the mutated strain. In contrast, the positive control, i.e. supernatant of the B. cereus DSM-31 strain showed a pronounced cytotoxicity even when applied at the lowest concentration on the Vero cells. Thus, the genetic modifications do not lead to a change of the status "non-cytotoxic".

### Example 6: Evaluation of the outgrowth behavior of the modified strain

The outgrowth behavior of the mutated strain was evaluated by measuring the beginning of metabolic activity of spore cultures. The redox dye containing resazurin was used as an indicator for metabolic activity, since the dye changes color upon reaction with reducing electron metabolites, which are produced after germination, in the beginning of the outgrowth phase. The increase in metabolic activity can be measured at 550 nm (Nagler et al. (2014); Appl Environ Microbiol 80(4):1314-1321; O'Brien et al. (2000); Eur J Biochem. 5421-6

## Claims

1. Endospore-forming Bacillota strain which exhibits improved outgrowth and/or germination characteristics.

2. Bacillota strain according to claim 1, wherein the strain possesses probiotic activity, in particular ability to inhibit pathogenic bacteria, preferably *C. perfringens,* ability to grow in presence of bile and/or ability to produce organic acids, preferably lactic acid.

3. Bacillota strain according to claim 1 or 2, wherein the improved outgrowth and/or germination characteristics are due to an enhanced activity of the germination receptor GerK.

4. Bacillota strain according to any of the preceding claims, wherein the improved outgrowth and/or germination characteristics are due to at least one mutation, preferably 1 to 10 mutations, more preferably 1 to 5 mutations, above all 2 to 4 mutations, in at least one subunit of the germination receptor GerK, wherein the subunit is preferably the spore germination protein KC (GerKC).

5. Bacillota strain according to any of the preceding claims, wherein the spore germination protein KC contains glycine at position 11 and/or serine at position 72.

6. Bacillota strain according to any of the preceding claims, wherein the gerKC gene has a sequence according to SEQ ID NO: 3 and/or the GerKC protein has a sequence according to SEQ ID NO: 4.

7. Bacillota strain according to any of the preceding claims, wherein the Bacilllota strain is a strain of the genus Bacillus and preferably selected from the species *B*. *subtilis, B. amyloliquefaciens, B. velezensis, B. licheniformis, B. paralicheniformis, B. pumilus, B. megaterium, B. lentus, B. laterosporus, B. alevi, B. cereus, B. badius, B. thurigiensis, B. coagulans, B. siamensis, B. glycinifermentans, B. methylotrophicus, B. thuringensis, B. polyfermenticus, B. vallismortis, B. tequilensis, B. atrophaeus, B. mojavensis, B. sonorensis, B. inaquosus* and *B. safensis,* more preferably from the species *Bacillus subtilis, Bacillus velezensis, Bacillus amyloliquefaciens, Bacillus pumilus* or *Bacillus megaterium,* above all from the species *Bacillus amyloliquefaciens or Bacillus velezensis.*

8. Bacillota strain according to any of the preceding claims, wherein the strain is a *B. velezensis* strain and wherein the genomic DNA of the strain is at least 99 %, preferably at least 99.5 % or 99.9 %, above all at least 99.99 %, identical to the genomic DNA of the strain CECT 5940.

9. Feed or food additive containing a Bacillota strain according to any of the preceding claims.

10. Feed or food composition containing a Bacillota strain according to any of claims 1 to 8 or a feed or food additive according to claim 9 and preferably at least one further feed or food ingredient selected from carriers, proteins, carbohydrates, fats, further probiotics, prebiotics, enzymes, vitamins, immune modulators, milk replacers, minerals, amino acids, carriers, coccidiostats, acid-based products and/or medicines, such as antibiotics.

11. Pharmaceutical composition containing a Bacillota strain according to any of claims 1 to 8 and a pharmaceutically acceptable carrier.

12. Use of a Bacillota strain according to any of claims 1 to 8 or of a feed or food additive according to claim 9 in the preparation of a feed or food composition

13. Use of a Bacillota strain according to any of claims 1 to 8 in the preparation of a pharmaceutical composition, preferably in the preparation of a pharmaceutical composition for treating, preventing or mitigating the course of a gut disease.

14. Use of a Bacillota strain according to any of claims 1 to 8 and/or of a feed or food additive according to claim 9 and/or of a feed or food composition according to claim 10 for preparing a composition for enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals.

15. Method of feeding animals, wherein the animals are fed with a Bacillota strain according to any of claims 1 to 8 and/or with a feed or food additive according to claim 9 and/or with a feed composition according to claim 11.

16. GerKC protein with a sequence identity of at least 95 %, preferably at least 98 or 99 %, to the GerKC sequence of the strain *B. velezensis* CECT 5940 as disclosed in SEQ ID NO: 2, wherein the protein sequence comprises at least one, preferably both, mutations selected from T31G and C214T.
